(19) Europäisches Patentamt
European Patent Office
Office européen des brevets

(11) **EP 1 616 583 B1**

(12) **EUROPÄISCHE PATENTSCHRIFT**

(45) Veröffentlichungstag und Bekanntmachung des Hinweises auf die Patenterteilung:
**25.06.2008 Patentblatt 2008/26**

(51) Int Cl.:
*A61L 15/58* (2006.01)   *A61L 15/60* (2006.01)

(21) Anmeldenummer: 04016789.2

(22) Anmeldetag: **16.07.2004**

(54) **Heissschmelzzusammensetzung mit Hydrocolloid**

Hot-melt composition comprising a hydrocolloid

Composition thermofusible comprenant des hydrocolloides

(84) Benannte Vertragsstaaten:
**CH DE GB LI**

(43) Veröffentlichungstag der Anmeldung:
**18.01.2006 Patentblatt 2006/03**

(73) Patentinhaber: **Collano AG**
**6203 Sempach-Station (CH)**

(72) Erfinder:
• **Frei, Pia**
  **6221 Rickenbach (CH)**
• **Dobmann, Andreas**
  **6208 Oberkirch (CH)**
• **Nyffeler, Judith**
  **6204 Sempach-Stadt (CH)**

(74) Vertreter: **Müller, Christoph Emanuel et al**
**Hepp, Wenger & Ryffel AG**
**Friedtalweg 5**
**9500 Wil (CH)**

(56) Entgegenhaltungen:
WO-A-20/04083302    WO-A-20/04098668
DE-A- 2 631 277    DE-A- 3 438 811
DE-C- 10 008 842    US-A- 4 551 490
US-A- 4 738 257    US-A- 6 025 071
US-A1- 2003 060 112    US-B1- 6 190 689
US-B1- 6 326 524

• BASF: "polymerforschung - der Weg zur Innovation URL - http://www.basf.de/de/corporate/innovation en/fakten/polymerforschung /Weg zur Inn DWWW-2003-07-31" BASF-GRUPPE FAKTEN ZUR FORSCHUNG POLYMERFORSCHUNG, 31. Juli 2003 (2003-07-31), XP002249742

**Beschreibung**

Die Erfindung betrifft eine Hot-melt Zusammensetzung, ein Verfahren zur Bereitstellung und/oder Verarbeitung einer solchen Hot-melt Zusammensetzung, ein Verfahren zur Verringerung des Auftretens von Verfärbungen bei der Bereitstellung und/oder Verarbeitung einer solchen Hot-melt Zusammensetzung, einem Verfahren zur insbesondere vollständigen Vernetzung von Prepolymeren in einer solchen Hot-melt Zusammensetzung, einem Wundverband umfassend eine solche Hot-melt Zusammensetzung, sowie der Verwendung von insbesondere bestrahlungs-induziert vernetzbaren Prepolymeren gemäss den Oberbegriffen der unabhängigen Patentansprüche.

Hydrocolloid-Haftklebstoffe gewinnen derzeit in der Wundversorgung zunehmend an Bedeutung. Hierbei dienen die eingesetzten Hydrocolloid-Haftklebstoffe sowohl zur mechanischen Stabilisierung der Wunde wie auch als Absorber für die beim Heilungsprozess austretenden Wundsekrete. Die notwendigen Hydrocolloide sind hierbei in einer kontinuierlichen Phase ("Matrix") aus thermoplastischem Kautschuk eingebettet. Die Formulierungen sind typischerweise und bevorzugt so eingestellt, dass die bis zu 5-fache Gewichts-Menge an Wasser bzw. Wundsekret aufgenommen werden kann, ohne dass die Integrität der Matrix verloren geht. Zudem gibt der medizinische Bereich Vorgaben bzgl. der notwendigen Hauthaftung vor, die erfüllt werden müssen.

Die heute in der Praxis eingesetzten thermoplastischen Kautschuke sind typischerweise SIS- (Styrol-Isopren-Styrol)oder SBS-(Styrol-Butadien-Styrol) Blockcopolymere. Hierin sind polymere Verbindungen mit hoher Wasserabsorptionsfähigkeit eingelassen, typischerweise in Pulverform. Diese nehmen im Lauf des Heilungsprozesses das aus der Wunde austretende Sekret auf und quellen dabei auf. Daraus ergeben sich hohe Anforderungen an die Kohäsionskraft der Matrix, die auch im aufgequollten Zustand der Hydrocolloide ihre mechanische Integrität nicht verlieren darf. Um diese Anforderungen zu erfüllen, müssen zwangsläufig thermoplastische Kautschuke mit hohem Molekulargewicht verwendet werden, was in Kombination mit dem absorbierenden Hydrocolloid zu hohen Schmelzviskositäten der Zusammensetzung führt.

Der Stand der Technik ist umfassend beschrieben von Roger Lipman in Medical Device & Diagnostic Industry Magazine 1999, "Hydrocolloid PSAs: New Formulation Strategies".

Typischerweise besitzen die bekannten Zusammensetzungen eine Schmelviskosität im Bereich von 130°C bis 150°C von weit über 300 Pa*S, zumeist gar von über 500 Pa*s (Messmethode Kegel/Platte gemäss DIN EN ISO 3219). Hierbei ist es nachteilig, dass die Zusammensetzungen daher nur mittels Kalandertechnologie unter hohen Drücken und Temperaturen verarbeitet, bspw. auf ein Trägermaterial aufgebracht werden können. Weithin verbreitete Hot-melt Verarbeitungsanlagen sind hingegen zur Verarbeitung derartiger Zusammensetzungen aufgrund der hohen Schmelzviskosität nicht geeignet. Typischerweise ist die Verarbeitung in Hot-melt Verarbeitungsanlagen wie bspw. Breitschlitzdüsen, Walzen etc. bereits bei Schmelzviskositäten von 100 Pa*s erheblich erschwert; niedrigere Schmelzviskositäten sind daher bei der Verarbeitung in derartigen Anlagen bevorzugt.

Zudem wird häufig eine unerwünschte, aber unvermeidliche Verfärbung der Zusammensetzungen beobachtet, wenn die hohe Schmelzviskosität der Zusammensetzungen des Stands der Technik durch höheres Erhitzen umgangen wird. Auch können eine Vielzahl von Hydrocolloiden wie bspw. Cellulosen, Pektinen nicht sehr hoch erhitzt werden (typischerweise nur sehr kurzfristig über ca. 100°C), was einer Erniedrigung der Schmelzviskosität über eine Erhöhung der Verarbeitungstemperatur ebenfalls enge Grenzen setzt.

US 4,738,257 beschreibt einen Wundverband mit einer Schicht aus Polyisobutylen und Ethylenvinylacetat-Harz. In diese Mischung sind Hydrocolloide eingelassen. Mittels ionisierender Strahlung (Elektrohnenstrahl, EP) erfolgt während der Sterilisierung eine Vernetzung des Ethylvinylacetat-Harzes. Nachteilig ist auch bei diesem Verfahren die Notwendigkeit, aufgrund der zu hohen Schmelzviskosität bei niedrigen Temperaturen, insbesondere ca. 130°C bis 150°C, eine Verarbeitung bspw. mittels Kalandern vornehmen zu müssen; die Zusammensetzungen sind mit konventionellen Hot-melt Auftragseinrichtungen insbesondere bei Temperaturen von ca. 130°C bis 150°C nicht verarbeitbar. Zudem ist aufgrund der zumeist notwendigen Schichtdicke von typischerweise 0.5 mm bis 1 mm mit einer nur unvollständigen Vernetzung des Ethylvinylacetat-Harzes mittels ionisierender Strahlung auszugehen, resultierend in mangelhafter Reproduzierbarkeit und mangelhafter Qualitätskonstanz.

Es ist daher eine Aufgabe der Erfindung, die Nachteile des bekannten zu vermeiden, insbesondere also eine Hot-melt Zusammensetzung mit Hydrocolloiden bereitzustellen, vorzugsweise zur Verwendung als Wundverband, welche bei niedrigeren Temperaturen eine derartige Schmelzviskosität aufweist, dass eine Verarbeitbarkeit mit konventionellen Hot-melt Auftragseinrichtungen ermöglicht ist. Darüber hinaus ist es eine Aufgabe der Erfindung, die Verwendung einer derartigen Zusammensetzung im medizinischen Bereich zu ermöglichen sowie möglichst mit bekannten Hydrocolloid-Haftklebstoffen vergleichbare Resultate hinsichtlich der Haftung, der Fähigkeit zur Aufnahme von Wundflüssigkeit, sowie der dauerhaften Integrität der Matrix zu erzielen.

Diese Aufgabe wird mit einer Hot-melt Zusammensetzung, einem Verfahren zur Bereitstellung und/oder Verarbeitung einer solchen Hot-melt Zusammensetzung, einem Verfahren zur Verringerung des Auftretens von Verfärbungen bei der Bereitstellung und/oder Verarbeitung einer solchen Hot-melt Zusammensetzung, einem Verfahren zur insbesondere vollständigen Vernetzung von Prepolymeren in einer solchen Hot-melt Zusammensetzung, einem Wundverband umfassend eine solche Hot-melt Zusammensetzung, sowie der Verwendung von insbesondere bestrahlungs-induziert vernetzbaren Prepolymeren gemäss den kennzeichnenden Teilen der unabhängigen Patentansprüche. Weitere Aspekte der Aufgabe werden dem Fachmann im Rahmen der Beschreibung und der Ausführungsbeispiele offenbar.

Eine erfindungsgemässe Hot-melt Zusammensetzung beinhaltet

(a) eine chemisch vernetzte Polymermatrix; sowie

(b) in der chemisch vernetzten Polymermatrix eingebettete Hydrocolioide;

welche erhältlich ist durch Verarbeitung einer Schmelze in einem Hot-melt Verfahren, wobei die Schmelze eine Schmelzviskosität von weniger als 100 Pa*s, vorzugsweise von weniger als 75 Pa*s, besonders bevorzugt von weniger als 50 Pa*s aufweist bei einer Verarbeitungstemperatur von weniger als 150°C, vorzugsweise von weniger als 140°C, besonders bevorzugt von weniger als 130°C, und Vernetzung der Prepolymeren mittels Photopolymerisation.

Unter einer Hot-melt Zusammensetzung werden hier und im folgenden bei Raumtemperatur fest, wasser- und lösungsmittelfreie Zusammensetzungen, insbesondere Klebstoffe, vorzugsweise Haftklebstoffe (hot melt pressure sensitive adhesives, HMPSAs) verstanden, welche aus der Schmelze verarbeitet, insbesondere auf ein Trägermaterial aufgebracht werden, und welche beim Abkühlen unter Verfestigung physikalisch abbinden.

Die Schmelzviskositäten sind hier und im folgenden bestimmt gemäss DIN EN ISO 3219, Messmethode Kegel/Platte.

Durch die Bereitstellung einer Schmelze mit einer Schmelzviskosität von weniger als 100 Pa*s, vorzugsweise von weniger als 75 Pa*s, besonders bevorzugt von weniger als 50 Pa*s bei einer Verarbeitungstemperatur von weniger als 150°C, vorzugsweise von weniger als 140°C, besonders bevorzugt von weniger als 130°C ist es im Rahmen der Erfindung ermöglicht, konventionelle Hot-melt Auftragsvorrichtungen wie bspw. ausgestattet mit Breitschlitzdüsen oder Walzen zur Verarbeitung der Schmelze zu verwenden. Zudem wird durch die Bereitstellung einer solchermassen charakterisierten Schmelze die Notwendigkeit von hohen Temperaturen, insbesondere von Temperaturen von mehr als 150°C während der Verarbeitung vermieden.

Gemäss der Erfindung ist die chemisch vernetzte Polymermatrix aus bestrahlungs-induziert vernetzten Prepolymeren gebildet. Geeignete Prepolymere sind hierbei in ihrem Molekulargewicht vorzugsweise derart ausgewählt, dass sie der resultierenden Hot-melt Zusammensetzung insbesondere nach der Vernetzung Festigkeit bei Raumtemperatur verleihen, jedoch während der Verarbeitung, insbesondere dem Auftrag auf ein Trägermaterial, der Schmelze eine für konventionelle Hot-melt Auftragsvorrichtungen geeignete Viskosität verleihen. Typische Schmelzviskositäten sind hierbei Schmelzviskositäten betragen weniger als 100 Pa*s, vorzugsweise weniger als 75 Pa*s, besonders bevorzugt weniger als 50 Pa*s bei einer Verarbeitungstemperatur von weniger als 150°C, vorzugsweise von weniger als 140°C, besonders bevorzugt von weniger als 130°C.

Geeignete bestrahlungs-induziert vernetzbare Prepolymere weisen (latente oder freie) reaktive Funktionalitäten auf, die mittels Photopolymerisation vernetzbar sind.

Gemäss einem im Rahmen der Erfindung besonders bevorzugten Ausführungsbeispiel werden als Prepolymere UV-vernetzende Acrylate in der Hot-melt Zusammensetzung eingesetzt. Durch Bestrahlung mit energiereichem Licht, insbesondere UV-Licht im Wellenlängenbereich von ca. 200 nm bis 450 nm, kann eine Vernetzung der Prepolymere bewirkt werden. Besonders bevorzugte UV-vernetzende Acrylat-Prepolymere sind solche der Produktfamilie acResin® von der Firma BASF, wie bspw. acResin 203 UV oder 204 UV. Auch Kombinationen solcher Polymere sind selbstverständlich möglich. Diese Substanzen zeichen sich durch einpolymerisierte Monomere aus, an die unter Verwendung einer Spacergruppe UV-aktivierbare Photoinitiatorgruppen chemisch gebunden sind. Der Zusatz niedermolekularer Photoinitatoren - insbesondere für medizinische Zwecke ohnehin zu vermeiden - ist somit zur Erzielung einer ausreichenden und raschen Vernetzung nicht notwendig.

Selbstverständlich ist es im Rahmen der Erfindung ebenfalls möglich, übliche Photoinitiatoren in der Hot-melt Zusammensetzung vorzusehen, welche ein beschleunigtes Einsetzen der Vernetzung des Prepolymers bewirken. Solchermassen geeignete Photoinitiatoren sind bspw. Acetophenon, Benzoinether, Benzyldialkylketole, oder deren Derivate. Der Gehalt an Photoinitiator ist typischerweise gering, vorzugsweise 0.05 bis 10 Gew.-Teile, besonders bevorzugt lediglich 0.1 bis 2 Gew.-Teile der Hot-melt Zusammensetzung. Insbesondere für medizinische Anwendungen ist es besonders bevorzugt, keine freien, niedermolekularen Photoinitiatoren vorzusehen; es können daher besonders bevorzugt einpolymerisierte Photoinitiatoren, bspw. ethylenisch ungesättigte Verbindungen mit einer Photoinitiatorgruppe vorgesehen sein, bspw. in einem Anteil von 0.05 bis 10 Gew.-Teilen, bevorzugt von 0.1 bis 2 Gew.-Teilen, besonders bevorzugt von 0.1 bis 1 Gew.-Teil der Hot-melt Zusammensetzung. Derartige Verbindungen sind bspw. bekannt aus EP-A-346 734, EP-A-377 199 (Anspruch 1), DE-A-40 37 079 (Anspruch 1) oder DE-A-38 44 444 (Anspruch 1); die Offenbarung der genannten Dokumente wird bezüglich dieser Photoinitiatoren hiermit zum Bestandteil dieses Dokuments erklärt.

In einer weiteren Ausführungsform der Erfindung weist die Hot-melt Zusammensetzung Prepolymere in einem Anteil von etwa 30 Gew.-% bis etwa 80 Gew.-%, bevorzugt von etwa 40 Gew.-% bis etwa 50 Gew.-% auf. Derartige Anteile von Prepolymeren haben sich als zweckmässig erwiesen, um eine auch in aufgequollenem Zustand mechanisch stabile Polymermatrix zu bilden. Hierfür werden die Prepolymere nach der Hot-melt Verarbeitung vernetzt.

Die Hot-melt Zusammensetzung gemäss der Erfindung weist Hydrocolloide auf, welche in der Polymermatrix eingebettet sind, und ausgewählt sind aus der Gruppe bestehend aus Superabsorbern, insbesondere acrylat-basierten Superabsorbern, carboxylierten Cellulosen, Pektinen, Alginaten, Vinylpolymeren, Acrylatpolymeren, Ethylenoxidpolymeren. Besonders bevorzugt sind im Rahmen der Erfindung Superabsorber, insbesondere acrylat-basierte Superabsorber. Diese sind thermisch stabiler als beispielsweise Cellulosen oder Pektine, welche nur sehr kurzfristig ohne Verfärbung und/oder Zersetzung thermischer Belastung von bis zu 130°C, insbesondere bis zu 150°C widerstehen. Superabsorber hingegen sind thermisch bis in diesen Bereich belastbar, ohne Verfärbung und/oder Zersetzung zu zeigen. Hydrocolloide mit möglichst niedrigen, mittleren Partikelgrössen, bereitgestellt in Pulverform sind im Rahmen der Erfindung bevorzugt. Im Rahmen der Erfindung derzeit bevorzugt verwendet wird der Superabsorber T 5066 F der Firma Degussa (Stockhausen), mit einer Partikelgrösse von 0-63 $\mu$m, beinhaltend weniger als 2 Gew.-% Partikeln mit einer Grösse von mehr als 63 $\mu$m. Selbstverständlich können im Rahmen der Erfindung auch die thermisch weniger stabilen Hydrocolloide wie Pektine und Cellulosen verwendet werden, falls die weiteren Komponenten der Zusammensetzung, insbesondere die Prepolymere derart ausgewählt werden, dass eine für gängige Hot-Melt Verarbeitungsanlagen geeignete Schmelzviskosität der Zusammensetzung auch bereits unterhalb der für das jeweilige Hydrocolloid kritischen Temperatur erzielbar ist.

Es ist besonders bevorzugt, dass die Hot-melt Zusammensetzung die Hydrocolloide in einem Anteil von etwa 10 Gew.-% bis etwa 40 Gew.-%, bevorzugt von etwa 15 Gew.-% bis etwa 25 Gew.-% umfasst. Die Fähigkeit zur Wasseraufnahme steigt mit dem Anteil an Hydrocolloid; hohe Anteile an Hydrocolloiden sind daher vorteilhaft, beanspruchen jedoch in aufgequollenem Zustand die Polymermatrix stark. Mit einer erfindungsgemässen Zusammensetzung, beinhaltend eine Polymermatrix aus nachträglich vernetzten Prepolymeren, sind nunmehr ähnlich hohe Anteile an Hydrocolloiden in derartigen Zusammensetzungen mit konventionellen Hot-melt Verarbeitungsanlagen realisierbar, wie sie bis anhin lediglich bspw. mit der Kalandertechnik verarbeitet werden konnten.

In weiteren bevorzugten Ausführungsformen beinhaltet die Hot-melt Zusammensetzung weiter:

a) einen insbesondere kohlenwasserstoff-basierten Klebrigmacher, vorzugsweise umfassend einen Anteil von etwa 2 Gew.-% bis etwa 20 Gew.-%, besonders bevorzugt von etwa 5 Gew.-% bis etwa 10 Gew.-%; und/oder

b) einen insbesondere adipinsäure-basierten Weichmacher, vorzugsweise umfassend einen Anteil von etwa 5 Gew.-% bis etwa 20 Gew.-%, besonders bevorzugt von etwa 10 Gew.-% bis etwa 15 Gew.-%; und/oder

c) Stabilisator(en), vorzugsweise umfassend einen Anteil von bis zu etwa 1 Gew.-%, besonders bevorzugt von bis zu etwa 0,5 Gew.-%.

Durch die nachträgliche Vernetzung der Prepolymere steigt das durchschnittliche Molekulargewicht der Polymermatrix, wodurch ein Anstieg der kohäsiven Kräfte bewirkt ist (Verbesserung der mechanischen Integrität der Matrix auch in aufgequollenem Zustand); dies führt jedoch parallel zu einer Reduktion der Klebrigkeit. Überraschend kann jedoch, trotz der bereits hohen Anteile von Hydrocolloid und Prepolymer (bzw. Polymermatrix), die Klebrigkeit durch Zusatz von geeigneten, insbesondere kohlenwasserstoff-basierten Klebrigmachern wieder erhöht werden. Insbesondere Copolymere aus Vinylmethylether und Maleinsäure und/oder Copolymere aus Vinylmethylether und Pyrollidon sind hierfür besonders bevorzugt, insbesondere bereitgestellt als Anhydrid. Besonders bevorzugt sind derzeit insbesondere die Copolymere der Gantrez® Produktfamilie, insbesondere die Gantrez S Produktfamilie der Firma ISP, International Speciality Products, Wayne, New Jersey 07470 USA.

Als Weichmacher sind derzeit insbesondere adipinsäure-basierte Weichmacher wie bspw. Plastomoll DNA der Firma BASF bevorzugt. Die Auswahl eines geeigneten Weichmachers durch den Fachmann unterliegt jedoch im weiteren keinen Beschränkungen, so lange diese kompatibel mit den Anforderungen des Verwendungszwecks insbesondere im medizinischen Bereich sind und eine Verarbeitung in konventionellen Hot-melt Auftragsvorrichtungen erlauben.

Falls aufgrund des Anwendungszwecks notwendig oder erwünscht, so können der Zusammensetzung auch Stabilisatoren (bspw. aus der Irganox-Produktfamile, z.B. Irganox B612 der Firma Ciba Spezialitätenchemie AG) in insbesondere geringen Anteilen zugesetzt werden. Die Auswahl eines geeigneten Stabilisators durch den Fachmann unterliegt jedoch im weiteren keinen Beschränkungen, so lange dieser kompatibel mit den Anforderungen des Verwendungszwecks insbesondere im medizinischen Bereich ist und eine Verarbeitung in konventionellen Hot-melt Auftragsvorrichtungen erlauben.

Bevorzugt beinhaltet die Hot-melt Zusammensetzung weiter einen die dynamische Wasseraufnahmefähigkeit verbessernden Zusatzstoff:

- bevorzugt: einen sulfonierten Copolyester, vorzugsweise in einem Anteil von etwa 5 Gew.-% bis etwa 30 Gew.-%,

besonders bevorzugt in einem Anteil von etwa 10 Gew.-% bis etwa 15 Gew.-%; und/oder

- eine Fluorcarbonverbindung, vorzugsweise in einem Anteil von etwa 0,5 Gew.-% bis etwa 2 Gew.-%, besonders bevorzugt in einem Anteil von etwa 0,5 Gew.-% bis etwa 1 Gew.-%.

Insbesondere sulfonierte Copolyester, bspw. aus der AQ-Produktfamilie von Eastman Chemicals, z.B. AQ 1045, verbessern die dynamische Wasseraufnahmefähigkeit erheblich, was insbesondere in einer Vielzahl medizinischer Anwendungen von Vorteil ist.

Die Erfindung betrifft weiter ein Verfahren zur Bereitstellung und/oder Verarbeitung einer Hot-melt Zusammensetzung insbesondere gemäss Anspruch 1, vorzugsweise einer Hydrocolloid-Haftklebstoff-Formulierung, umfassend die folgenden Schritte:

a) Beigabe mindestens eines vernetzbaren Prepolymers ei-ner derartigen Beschaffenheit und in einer derartigen Menge zu der Zusammensetzung, so dass eine Schmelzviskosität von weniger als 100 Pa*s, vorzugsweise von weniger als 75 Pa*s, besonders bevorzugt von weniger als 50 Pa*s, bei einer Verarbeitungstemperatur von weniger als 150°C, vorzugsweise von weniger als 140°C, besonders bevorzugt von weniger als 130°C erzielt wird;
b) Verarbeitung der Zusammensetzung in einem Hot-melt Verfahren;
c) Vernetzung des Prepolymers der Zusammensetzung mittels Photopolymerisation.

Im Rahmen dieses Verfahrens ist es besonders bevorzugt, dass die Verarbeitung in einem Hot-melt Verfahren das Auftragen auf ein Release-Material und eine Trägerfolie in einem sog. TransferVerfahren umfasst. Hierbei erfolgt der Auftrag in einem Hot-melt Verfahren zunächst auf ein Release-Material, insbesondere eine silikonisierte, biaxial orientierte Polypropylen-Folie (BOPP-Folie), wobei nachträglich eine Laminierung mit einer Trägerfolie erfolgt. Derzeit werden polyurethan-basierte Trägerfolien verwendet. Besonders vorteilhaft wird das Release-Material und ggf. auch die Trägerfolie derart ausgewählt, dass sie für eine nachträglich auf die Zusammensetzung, insbesondere die Prepolymere der Zusammensetzung einwirkende Strahlung, wie bspw. UV-Strahlung, im wesentlichen durchlässig ist. Dies ermöglicht eine Bestrahlung der Zusammensetzung auch durch die Trägerfolie und/oder das Release-Material hindurch. Vorzugsweise erfolgt eine Bestrahlung (ein- oder zweiseitig) im Rahmen der Erfindung in-line mit dem Hot-melt Verfahren, also im wesentlichen direkt nach dem Auftrag der Schmelze auf das Release-Material und vor der Laminierung mit einer Trägerfolie in einer konventionellen Hot-melt Verarbeitungsanlage.

Vorzugsweise wird die Zusammensetzung nach der Verarbeitung in einem Hot-melt Verfahren, insbesondere beinhaltend das Auftragen auf ein Release-Material, anschliessend, vorzugsweise ebenfalls in-line mit dem Hot-melt Verfahren, die Abdeckung und Laminierung der Zusammensetzung mit einer insbesondere polyurethan-basierten Trägerfolie. Das Release-Material erleichtert die Bereitstellung der Hot-melt Zusammensetzung, bspw. für medizinische Zwecke: Das Release-Material kann mit geringem manuellen Aufwand abgezogen werden, und die Hot-melt Zusammensetzung liegt frei und kann appliziert werden, bspw. als Wundverband.

Vorzugsweise erfolgt die Verarbeitung der Zusammensetzung in einem Hot-melt Verfahren bei einer Temperatur der Schmelze von max. 150°C, vorzugsweise von max. 140°C, besonders bevorzugt von max. 130°C erfolgt. Selbstverständlich ist es im Rahmen der Erfindung ebenfalls möglich, die Hot-melt Verarbeitung bei niedrigeren Temperaturen durchzuführen, falls bei niedrigeren Temperaturen bereits aufgrund der spezifischen Formulierung, insbesondere den ausgewählten Prepolymeren, eine ausreichend niedrige Schmelzviskosität von max. 100 Pa*s gegeben ist.

Besonders bevorzugt wird die Vernetzung der Propolymere der Zusammensetzung nach der Hot-melt Verarbeitung, insbesondere in-line, durch Bestrahlung insbesondere mit energiereichem UV-Licht (bspw. im Wellenlängenbereich von etwa 200 nm bis etwa 450 nm) durch die Trägerfolie und/oder die Abdeckfolie hindurch vorgenommen bzw. induziert. Hierdurch kann ggf. bei grösseren Schichtdicken eines flächigen Substrats auch beidseitig bestrahlt werden, wodurch die Zuverlässigkeit der vollständigen Vernetzung und damit die Qualitätskonstanz entscheidend verbessert werden kann.

Die Erfindung betrifft des weiteren einen saugfähigen Medizinal- oder Kosmetikartikel, insbesondere einen Wundverband wie bspw. Pflaster, Kompressen, etc., umfassend eine vorstehend beschriebene Hot-melt Zusammensetzung, vorzugsweise hergestellt unter Benutzung eines der vorstehend beschriebenen Verfahren.

Die Erfindung betrifft darüber hinaus die Verwendung von insbesondere bestrahlungs-induziert vernetzbaren Prepolymeren

- zur Bereitstellung einer vorstehend beschriebenen Hot-melt Zusammensetzung; und/oder
- in einem der vorstehen beschriebenen Verfahren.

Die Erfindung betrifft weiters ein Verfahren zur Verringerung des Auftretens von Verfärbungen bei der Bereitstellung und/oder Verarbeitung einer Hot-melt Zusammensetzung, vorzugsweise einer Hydrocolloid-Haftklebstoff-Formulierung, wobei die Bereitstellung und/oder Verarbeitung der Hot-melt Zusammensetzung bei einer Temperatur der Schmelze von max. 150°C, vorzugsweise von max. 140°C, besonders bevorzugt von max. 130°C erfolgt. Durch eine Verarbeitung bis maximal in diesem Temperaturbereich treten in aller Regel keine sonst insbesondere bei Hydrocolloid-Hot-melt Zusammensetzungen beobachtbaren Verfärbungen der Zusammensetzung durch thermischen Stress auf. Insbesondere die Generierung der Polymermatrix durch nachträgliche Vernetzung der Prepolymere ermöglicht erst diese vergleichsweise tiefen Verarbeitungstemperaturen, so dass rein-weisse bzw. transparente Zusammensetzungen bereitgestellt werden können, welche neben einer verbesserten Ästhetik auch eine besser Verfolgbarkeit des Heilungsprozesses beispielsweise einer abgedeckten Wunde gewährleisten.

Die Erfindung wird nachfolgend anhand eines bevorzugten Ausführungsbeispiels erläutert.

Eine derzeit im Rahmen der Erfindung bevorzugte Rezeptur beinhaltet die folgenden Komponenten:

| | |
|---|---|
| 43,2 Gew.-% | acResin A204 UV, BASF (UV-vernetzbares Acrylat Prepolymer) |
| 13,5 Gew.-% | AQ 1045, Eastman (sulfonierter Copolyester) |
| 13,5 Gew.-% | Plastomoll DNA, BASF (Weichmacher) |
| 7,5 Gew.-% | Gantrez S 97 BF, ISP (Klebrigmacher) |
| 22 Gew.-% | Superabsorber T 5066 F, Degussa (Stockhausen) (Superabsorber) |
| 0,3 Gew.-% | Irganox B612, Ciba (Stabilisator) |

Die Komponenten werden innig gemischt. Die Zusammensetzung ist bei Rautemperatur sehr hochviskos. Die Zusammensetzung wurde wahlweise bei Temperaturen von 130°C, 140°C und 150°C auf eine silikonisierte, biaxial orientierte Polypropylen-Folie (BOPP-Folie, Release-Material) aufgetragen ($200\,g/m^2$) und mit einer polyurethan-basierte Trägerfolie (100 $\mu$m Dicke) laminiert mittels einer konventionellen Hot-melt Verarbeitungsanlage der Firma Nordson mit Breitschlitzdüse. Die Schmelzviskosität der Schmelze lag in allen Fällen weit unterhalb der für derartige Verarbeitungsanlagen kritischen Obergrenze von etwa 100 Pa*s; selbst bei 130°C betrug die Schmelzviskosität lediglich 25 Pa*s.

Das Auslösen der Vernetzung der Prepolymere erfolgte in-line, direkt nach der Hot-meit Verarbeitung durch Belichtung von beiden Seiten mit Belichtungsgeräten UV Minicure (Firma IST). Die Vernetzungsdosis beträgt $240\,mJ/cm^2$ im UV-C Bereich (250-260 nm). Es resultiert eine rohweisse Beschichtung.

Die statische Wasserabsorption einer isotonischen, wässrigen 0,9%igen NaCl-Lösung wurde nach 24 Stunden Lagerung der Zusammensetzung auf der Trägerfolie bei 37°C zu 250 Gew.-% bestimmt, bezogen auf eine Musterfläche von 19,6 $cm^2$. Die Berechnung erfolgt hierbei folgendermassen:

Absorption in $g/m^2/24h$:

```
(((Gewicht Muster nach 24h bei 37°C) - (Gewicht Muster vor Test-
beginn)) / 19,6 cm²) * 10'000 = Absorption in g/m²/24h
```

Absorption in %:

$$(100 / (\text{Gewicht Muster vor Testbeginn})) * (\text{Wasseraufnahme in g/24h}) = \text{Wasseraufnahme in } \%$$

Das Aussehen des Testmusters sowie der isotonischen Lösung wurden anschliessend visuell beurteilt:

| Integrität des Testmusters: | i.O. |
|---|---|
| Isotonische Testlösung: | transparent |
| Hot-melt Zusammensetzung: | weiss, kompakt |

Die Hauthaftung wurde in einem Paneltest überprüft:

| Soforthaftung: | gut |
|---|---|
| Haftung nach 1 Tag: | sehr gut |
| Haftung nach 3 Tagen: | sehr gut |
| Farbe / Aussehen nach 3 Tagen: | leicht trüb-transparent |
| Entfernbarkeit nach 3 Tagen: | schmerzfrei |

**Patentansprüche**

1. Zusammensetzung, beinhaltend

   (a) eine chemisch vernetzte Polymermatrix, erhältlich aus mittels Photopolymerisation vernetzbaren Prepolymeren; sowie
   (b) in der chemisch vernetzten Polymermatrix eingebettete Hydrocolloide;

   erhältlich durch

   - Verarbeitung einer Hot-melt Schmelze in einem Hot-melt Verfahren, wobei die Schmelze eine Schmelzviskosität von weniger als 100 Pa*s, vorzugsweise von weniger als 75 Pa*s, besonders bevorzugt von weniger als 50 Pa*s aufweist bei einer Verarbeitungstemperatur von weniger als 150°C, vorzugsweise von weniger als 140°C, besonders bevorzugt von weniger als 130°C; und
   - Vernetzung der Prepolymeren mittels Photopolymerisation.

2. Zusammensetzung gemäss Anspruch 1,
   **dadurch gekennzeichnet, dass** es sich um einen Hydrocolloid-Haftklebstoff handelt.

3. Zusammensetzung gemäss Anspruch 2,
   **dadurch gekennzeichnet, dass** die Prepolymere insbesondere UV-vernetzende Acrylate sind.

4. Zusammensetzung gemäss einem der Ansprüche 1 bis 3,
   **dadurch gekennzeichnet, dass** die Prepolymere einen Anteil von etwa 30 Gew.-% bis etwa 80 Gew.-%, bevorzugt von etwa 40 Gew.-% bis etwa 50 Gew.-% umfassen.

5. Zusammensetzung gemäss einem der Ansprüche 1 bis 4,
   **dadurch gekennzeichnet, dass** die Hydrocolloide ausgewählt sind aus der Gruppe bestehend aus Superabsorbern, insbesondere acrylat-basierten Superabsorbern, carboxylierten Cellulosen, Pektinen, Alginaten, Vinylpolymeren, Acrylatpolymeren, Ethylenoxidpolymeren.

6. Zusammensetzung gemäss Anspruch 5,
   **dadurch gekennzeichnet, dass** die Hydrocolloide einen Anteil von etwa 10 Gew.-% bis etwa 40 Gew.-%, bevorzugt von etwa 15 Gew.-% bis etwa 25 Gew.-% umfassen.

7. Zusammensetzung gemäss einem der Ansprüche 1 bis 6, weiter beinhaltend:

a) einen insbesondere kohlenwasserstoff-basierten Klebrigmacher, vorzugsweise umfassend einen Anteil von etwa 2 Gew.-% bis etwa 20 Gew.-%, besonders bevorzugt von etwa 5 Gew.-% bis etwa 10 Gew.-%; und/oder
b) einen insbesondere adipinsäure-basierten Weichmacher, vorzugsweise umfassend einen Anteil von etwa 5 Gew.-% bis etwa 20 Gew.-%, besonders bevorzugt von etwa 10 Gew.-% bis etwa 15 Gew.-%; und/oder
c) Stabilisator(en), vorzugsweise umfassend einen Anteil von bis zu etwa 1 Gew.-%, besonders bevorzugt von bis zu etwa 0,5 Gew.-%.

8. Zusammensetzung gemäss Anspruch 7,
**dadurch gekennzeichnet, dass** der Klebrigmacher Copolymere aus Vinylmethylether und Maleinsäure und/oder Copolymere aus Vinylmethylether und Pyrollidon umfasst.

9. Zusammensetzung gemäss einem der Ansprüche 1 bis 8, beinhaltend einen die dynamische Wasseraufnahmefähigkeit verbessernden Zusatzstoff:

• einen sulfonierten Copolyester, vorzugsweise in einem Anteil von etwa 5 Gew.-% bis etwa 30 Gew.-%, besonders bevorzugt in einem Anteil von etwa 10 Gew.-% bis etwa 15 Gew.-%; und/oder
• eine Fluorcarbonverbindung, vorzugsweise in einem Anteil von etwa 0,5 Gew.-% bis etwa 2 Gew.-%, besonders bevorzugt in einem Anteil von etwa 0,5 Gew.-% bis etwa 1 Gew.-%.

10. Verfahren zur Bereitstellung und/oder Verarbeitung einer Hot-melt Zusammensetzung gemäss Anspruch 1, vorzugsweise einer Hydrocolloid-Haftklebstoff-Formulierung, umfassend die folgenden Schritte:

a) Beigabe mindestens eines vernetzbaren Prepolymers einer derartigen Beschaffenheit und in einer derartigen Menge zu der Zusammensetzung, so dass eine Schmelzviskosität von weniger als 100 Pa*s, vorzugsweise von weniger als 75 Pa*s, besonders bevorzugt von weniger als 50 Pa*s, bei einer Verarbeitungstemperatur von weniger als 150°C, vorzugsweise von weniger als 140°C, besonders bevorzugt von weniger als 130°C erzielt wird;
b) Verarbeitung der Zusammensetzung in einem Hot-melt Verfahren;
c) Vernetzung des Prepolymers der Zusammensetzung mittels Photopolymerisation.

11. Verfahren gemäss Anspruch 10, wobei die Verarbeitung in einem Hot-melt Verfahren das Laminieren mit einer insbesondere Polyurethan-basierten Trägerfolie umfasst.

12. Verfahren gemäss einem der Ansprüche 10 oder 11, wobei die Verarbeitung in einem Hot-melt Verfahren den Auftrag der Zusammensetzung auf ein Release-Material, insbesondere ein silikonisiertes Release-Material, vorzugsweise eine silikonisierte, biaxial orientierte Polypropylen-Folie (BOPP-Folie) umfasst.

13. Verfahren gemäss einem der Ansprüche 10 bis 12,
umfassend den Schritt der Vernetzung des Prepolymers mittels UV-Bestrahlung.

14. Verfahren gemäss Anspruch 13,
umfassend den Schritt der Bestrahlung durch die Trägerfolie und/oder das Release-Material hindurch bzw. die doppelseitige Bestrahlung des sich auf dem Release-Material befindlichen Hot-Melts.

15. Saugfähiger Medizinal- oder Kosmetikartikel, insbesondere Wundverband, umfassend eine Zusammensetzung gemäss einem der Ansprüche 1 bis 9 und/oder hergestellt mit einem Verfahren gemäss einem der Ansprüche 10 bis 14.

16. Verwendung von bestrahlungs-induziert vernetzbaren Prepolymeren

• zur Bereitstellung einer Zusammensetzung gemäss einem der Ansprüche 1 bis 9; und/oder
• in einem Verfahren gemäss einem der Ansprüche 10 bis 14.

17. Verfahren zur Verringerung des Auftretens von Verfärbungen bei der Bereitstellung und/oder Verarbeitung einer Zusammensetzung gemäss Anspruch 1, vorzugsweise einer Hydrocolloid-Haftklebstoff-Formulierung,

**dadurch gekennzeichnet, dass** die Bereitstellung und/oder Verarbeitung der Hot-melt Zusammensetzung bei einer Temperatur der Schmelze von max. 150°C, vorzugsweise von max. 140°C, besonders bevorzugt von max. 130°C erfolgt.

**18.** Verfahren zur insbesondere vollständigen Vernetzung von Prepolymeren in einer insbesondere flächig auf ein Release-Material aufgetragenen und ggf. mit einer Trägerfolie laminierten Hot-melt Zusammensetzung gemäss Anspruch 1, vorzugsweise einer Hydrocolloid-Haftklebstoff-Formulierung,
**dadurch gekennzeichnet, dass** die Vernetzung mittels Bestrahlung, mittels Photopolymerisation, vorzugsweise mittels UV-Bestrahlung von beiden Seiten erfolgt, insbesondere durch die Trägerfolie und/oder das Release-Material hindurch.

**Claims**

**1.** A composition comprising

(a) a chemically crosslinked polymer matrix, obtainable from prepolymers crosslinkable by means of photopolymerization; and
(b) hydrocolloids embedded in the chemically crosslinked polymer matrix;

obtainable by

- processing a hot-melt melt in a hot-melt process, the melt having a melt viscosity of less than 100 Pa*s, preferably of less than 75 Pa*s, more preferably of less than 50 Pa*s, at a processing temperature of less than 150°C, preferably of less than 140°C, more preferably of less than 130°C; and
- crosslinking the prepolymers by means of photopolymerization.

**2.** The composition of claim 1, **characterized in that** the composition is a hydrocolloid pressure-sensitive adhesive.

**3.** The composition of claim 2, **characterized in that** the prepolymers are acrylates, in particular UV-crosslinking acrylates.

**4.** The composition of one of claims 1 to 3, **characterized in that** the prepolymers embrace a fraction of about 30% to about 80% by weight, preferably of about 40% to about 50% by weight.

**5.** The composition of one of claims 1 to 4, **characterized in that** the hydrocolloids are selected from the group consisting of superabsorbents, especially acrylate-based superabsorbents, carboxylated celluloses, pectins, alginates, vinyl polymers, acrylate polymers, and ethylene oxide polymers.

**6.** The composition of claim 5, **characterized in that** the hydrocolloids embrace a fraction of about 10% to about 40% by weight, preferably of about 15% to about 25% by weight.

**7.** The composition of one of claims 1 to 6, further comprising:

a) a tackifier, in particular a hydrocarbon-based tackifier, preferably embracing a fraction of about 2% to about 20% by weight, more preferably of about 5% to about 10% by weight; and/or
b) a plasticizer, in particular an adipic acid-based plasticizer, preferably embracing a fraction of about 5% to about 20% by weight, more preferably of about 10% to about 15% by weight; and/or
c) one or more stabilizers, preferably embracing a fraction of up to about 1% by weight, more preferably of up to about 0.5% by weight.

**8.** The composition of claim 7, **characterized in that** the tackifier embraces copolymers of vinyl methyl ether and maleic acid and/or copolymers of vinyl methyl ether and pyrollidone.

**9.** The composition of one of claims 1 to 8, comprising an additive which improves the dynamic water absorption capacity:

• a sulfonated copolyester, preferably in a fraction of about 5% to about 30% by weight, more preferably in a fraction of about 10% to about 15% by weight; and/or

• a fluorocarbon compound, preferably in a fraction of about 0.5% to about 2% by weight, more preferably in a fraction of about 0.5% to about 1% by weight.

10. A process for providing and/or processing a hot-melt composition of claim 1, preferably a hydrocolloid pressure-sensitive adhesive formulation, which comprises the following steps:

a) adding to the composition at least one crosslinkable prepolymer whose nature and amount are such as to give a melt viscosity of less than 100 Pa*s, preferably of less than 75 Pa*s, more preferably of less than 50 Pa*s, at a processing temperature of less than 150°C, preferably of less than 140°C, more preferably of less than 130°C;
b) processing the composition in a hot-melt process;
c) crosslinking the prepolymer of the composition by means of photopolymerization.

11. The process of claim 10, wherein the processing in a hot-melt process encompasses lamination with a backing film, in particular a polyurethane-based backing film.

12. The process of one of claims 10 or 11, wherein the processing in a hot-melt process encompasses the application of the composition to a release material, in particular a siliconized release material, preferably a siliconized, biaxially oriented polypropylene film (BOPP film).

13. The process of one of claims 10 to 12, comprising the step of crosslinking the prepolymer by means of UV irradiation.

14. The process of claim 13, comprising the step of irradiation through the backing film and/or through the release material and/or the double-sided irradiation of the hot-melt situated on the release material.

15. An absorbent medical or cosmetics article, in particular a wound dressing, comprising a composition of one of claims 1 to 9 and/or produced by a process of one of claims 10 to 14.

16. The use of prepolymers crosslinkable with induction by irradiation

• to provide a composition of one of claims 1 to 9; and/or
• in a process of one of claims 10 to 14.

17. A process for reducing the occurrence of discolorations in the provision and/or processing of a composition of claim 1, preferably a hydrocolloid pressure-sensitive adhesive formulation, **characterized in that** the provision and/or processing of the hot-melt composition takes place at a melt temperature of not more than 150°C, preferably of not more than 140°C, more preferably of not more than 130°C.

18. A process for crosslinking, in particular complete crosslinking, of prepolymers in a hot-melt composition of claim 1, preferably a hydrocolloid pressure-sensitive adhesive formulation, which is applied, in particular two-dimensionally, to a release material and is optionally laminated with a backing film, **characterized in that** the crosslinking takes place by means of irradiation, by means of photopolymerization, preferably by means of UV irradiation from both sides, in particular through the backing film and/or through the release material.

**Revendications**

1. Composition comprenant

(a) une matrice polymère réticulée chimiquement, pouvant être obtenue à partir de prépolymères réticulables par photopolymérisation ; ainsi que
(b) des hydrocolloïdes incorporés dans la matrice polymère réticulée chimiquement ;

pouvant être obtenue par

- traitement d'une masse fondue à chaud, dans un procédé de fusion à chaud, la masse fondue ayant une viscosité à chaud de moins de 100 Pa.s, de préférence de moins de 75 Pa.s, de façon particulièrement préférée de moins de 50 Pa.s à une température de traitement de moins de 150 °C, de préférence de moins de 140 °C de façon particulièrement préférée de moins de 130°C ; et
- réticulation des prépolymères par photopolymérisation.

2. Composition selon la revendication 1, **caractérisée en ce qu'**il s'agit d'un autoadhésif hydrocolloïde.

3. Composition selon la revendication 2, **caractérisée en ce que** les prépolymères sont en particulier des polyacrylates réticulables aux UV.

4. Composition selon l'une quelconque des revendications 1 à 3, **caractérisée en ce que** les prépolymères sont contenus en une proportion d'environ 30 % en poids à environ 80 % en poids, de préférence d'environ 40 % en poids à environ 50 % en poids.

5. Composition selon l'une quelconque des revendications 1 à 4, **caractérisée en ce que** les hydrocolloïdes sont choisis dans le groupe constitué par des superabsorbants, en particulier des superabsorbants à base d'acrylate, des celluloses carboxylées, des pectines, des alginates, des polymères vinyliques, des polymères d'acrylate, des polymères d'oxyde d'éthylène.

6. Composition selon la revendication 5, **caractérisée en ce** les hydrocolloïdes sont contenus en une proportion d'environ 10 % en poids à environ 40 % en poids, de préférence d'environ 15 % en poids à environ 25 % en poids.

7. Composition selon l'une quelconque des revendications 1 à 6, comprenant en outre :

   a) un agent d'adhésivité en particulier à base d'hydrocarbure, de préférence contenu en une proportion d'environ 2 % en poids à environ 20 % % en poids, de façon particulièrement préférée d'environ 5 % en poids à environ 10 % en poids ; et/ou
   b) un plastifiant en particulier à base d'acide adipique, de préférence contenu en une proportion d'environ 5 % en poids à environ 20 % en poids, de façon particulièrement préférée d'environ 10 % en poids à environ 15 % en poids ; et/ou
   c) un/des stabilisant(s), de préférence contenu(s) en une proportion allant jusqu'à environ 1 % en poids, de façon particulièrement préférée allant jusqu'à environ 0,5 % en poids.

8. Composition selon la revendication 7, **caractérisée en ce que** l'agent d'adhésivité comprend des copolymères d'oxyde de vinyle et de méthyle et d'acide maléique et/ou des copolymères de pyrrolidone et d'oxyde de vinyle et de méthyle.

9. Composition selon l'une quelconque des revendications 1 à 8, comprenant un additif améliorant la capacité dynamique d'absorption d'eau :

   • un copolyester sulfoné, de préférence en une proportion d'environ 5 % en poids à environ 30 % en poids, de façon particulièrement préférée en une proportion d'environ 10 % en poids à environ 15 % en poids ; et/ou
   • un composé fluorocarboné, de préférence en une proportion d'environ 0,5 % en poids à environ 2 % en poids, de façon particulièrement préférée en une proportion d'environ 0,5% en poids à environ 1% en poids.

10. Procédé pour la préparation et/ou le traitement d'une composition thermofusible selon la revendication 1, de préférence d'une composition d'autoadhésif hydrocolloïde, comprenant les étapes suivantes :

    a) addition à la composition d'au moins un prépolymère réticulable d'une nature telle et en une quantité telle qu'on atteint une viscosité à chaud de moins de 100 Pa.s, de préférence de moins de 75 Pa.s, de façon particulièrement préférée de moins de 50 Pa.s à une température de traitement de moins de 150 °C, de préférence de moins de 140 °C de façon particulièrement préférée de moins de 130°C ; et
    b) traitement de la composition dans un procédé de fusion à chaud ;
    c) réticulation par photopolymérisation du prépolymère de la composition.

11. Procédé selon la revendication 10, dans lequel le traitement dans un procédé de fusion à chaud comprend la stratification avec un film de support en particulier à base de polyuréthanne.

12. Procédé selon la revendication 10 ou 11, dans lequel le traitement dans un procédé de fusion à chaud comprend l'application de la composition sur un matériau antiadhésif, en particulier un matériau antiadhésif siliconé, de préférence un film de polypropylène siliconé, orienté biaxialement (film de BOPP) .

13. Procédé selon l'une quelconque des revendications 10 à 12, comprenant l'étape de la réticulation du prépolymère par irradiation UV.

14. Procédé selon la revendication 13, comprenant l'étape de l'irradiation à travers le film de support et/ou à travers le matériau antiadhésif ou l'irradiation des deux côtés du produit thermofusible se trouvant sur le matériau antiadhésif.

15. Article absorbant médical ou cosmétique, en particulier pansement pour le soin des plaies, comprenant une composition selon l'une quelconque des revendications 1 à 9 et/ou fabriqué par un procédé selon l'une quelconque des revendications 10 à 14.

16. Utilisation de prépolymères réticulables avec induction par irradiation

   • pour la préparation d'une composition selon l'une quelconque des revendications 1 à 9 ; et/ou
   • dans un procédé selon l'une quelconque des revendications 10 à 14.

17. Procédé pour diminuer l'apparition de colorations lors de la préparation et/ou du traitement d'une composition selon la revendication 1, de préférence d'une composition d'autoadhésif hydrocolloïde, **caractérisé en ce qu'**on effectue la préparation et/ou le traitement de la composition thermofusible à une température de la masse fondue d'au maximum 150 °C, de préférence d'au maximum 140 °C, de façon particulièrement préférée d'au maximum 130 °C.

18. Procédé pour la réticulation en particulier totale de prépolymères dans une composition thermofusible selon la revendication 1 en particulier appliquée à plat sur un matériau antiadhésif et éventuellement stratifiée avec un film de support, de préférence dans une composition d'autoadhésif hydrocolloïde, **caractérisé en ce que** la réticulation s'effectue par irradiation, par photopolymérisation, de préférence par irradiation UV des deux côtés, en particulier à travers le film de support et/ou à travers le matériau antiadhésif.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente

- US 4738257 A
- EP 346734 A
- EP 377199 A
- DE 4037079 A
- DE 3844444 A

### In der Beschreibung aufgeführte Nicht-Patentliteratur

- **ROGER LIPMAN.** Hydrocolloid PSAs: New Formulation Strategies. *Medical Device & Diagnostic Industry Magazine,* 1999